# FASCICULE DE BREVET EUROPEEN

(11) **EP 1 278 457 B1**
(45) Date de publication et mention de la délivrance du brevet: **26.10.2005**
(21) Numéro de dépôt: 01931805.4
(22) Date de dépôt: 04.05.2001
(51) Int. Cl.: A61B 5/0205, A61B 5/11, G08B 21/04

(54) **DISPOSITIF ET PROCEDE DE DETECTION DE SITUATIONS ANORMALES**
VERFAHREN UND VORRICHTUNG ZUR DETEKTION ABNORMALER SITUTATIONEN
DEVICE AND METHOD FOR DETECTING ABNORMAL SITUATIONS

(30) Priorité: 05.05.2000 FR 0005822
(43) Date de publication de la demande: 29.01.2003
(73) Titulaire: Universite de Rennes 1, 35065 Rennes (FR)
(72) Inventeur: GAGNARDE, Claude, F-56270 Ploemeur (FR); THUILLIER, Sandrine, F-56100 Lorient (FR); BILLON, Michel, F-22300 Lannion (FR); VALETTE, Michel, F-22560 Trebeurden (FR); COSQUER, Philippe, F-22560 Trebeurden (FR); VINESSE, Hélène, F-22300 Lannion (FR); LUTZLER, Pierre, F-77360 Vaires sur Marne (FR); FARALDI, Odile, 60880 Lemeux (FR); L'HER, Henry, F-22730 Tregastel (FR); SENTIEYS, Olivier, F-22700 Louannec (FR)
(74) Mandataire: Vidon, Patrice
(86) Numéro de dépôt international: PCT/FR2001/001375
(87) Numéro de publication internationale: WO 2001/085025

(56) Documents cités:
- EP-A- 0 877 346
- FR-A- 2 785 073
- GB-A- 2 312 309
- US-A- 5 515 858

## Description

Le domaine de l'invention est celui de la détection de certaines situations anormales, notamment pathologiques, et en particulier de chutes, chez un sujet humain. Une telle détection a notamment pour but la transmission d'une alarme vers une tierce personne (personne physique ou services,...) réalisant une fonction de télésurveillance.

L'invention peut trouver des applications dans de nombreuses situations, et peut notamment équiper des personnes âgées et/ou à mobilité réduite, des travailleurs isolés, des enfants, des animaux,...

Les systèmes de téléalarme connus sont généralement constitués d'un ensemble porter par le sujet et reliés à une base fixe, par exemple par une liaison HF. En cas de situation anormale, la base fixe émet un message codé, véhiculé par exemple par le réseau téléphonique, vers un centre spécialisé. Dans certaines institutions, le message peut être véhiculé par un réseau interne.

Dans tous les cas, ces systèmes prévoient un déclenchement à posteriori et volontaire du processus, suite à une chute ou à un malaise. Ceci suppose donc que le sujet ait la capacité et la volonté de le faire.

On connaît déjà différents systèmes de téléalarme. Notamment, il existe des dispositifs portés sous forme de médaillon autour du cou, qui nécessitent une action de serrage ou de traction pour émettre une alarme. Outre le fait qu'il s'agit de systèmes passifs (nécessitant une action volontaire du porteur), ces dispositifs produisent souvent de fausses alarmes, par exemple la nuit, quand ils sont portés et s'accrochent intempestivement dans la literie. De plus, on ne sait pas quand ils ne sont pas portés.

On connaît également des capteurs de mouvements, qui renseignent sur l'activité nocturne anormale, ou des systèmes de capteurs de position équipant les lits dans certains services médicaux.

En fait, pour décrire une chute à partir du centre de gravité d'une personne, il faut pouvoir établir la cinématique, c'est-à-dire connaître les conditions initiales (masse, taille, position), les trois accélérations et les trois rotations. Une telle solution est proposée dans le document de brevet français FR-2760116 qui utilise deux gyromètres. La trajectoire est calculée, et un inclinomètre informe de la fin du mouvement.

Il peut être porté dans une poche, mais il présente une consommation importante, ce qui est un facteur de limitation de son utilisation pour une personne en site isolé. De plus, il ne permet de détecter que des chutes importantes, est non une "micro chute" sur le lit du patient, ou une chute lente, par exemple le long d'un mur. Or on sait qu'une personne dépendante, physiquement limitée dans ses déplacements, cherche des appuis le long de son parcours. De même en position assise ou allongée, un malaise ne sera pas suivi d'une alarme.

On connaît encore les systèmes associant la détection de la décélération lors d'une chute et un canal vocal bidirectionnel, des systèmes associant la protection par rapport à la verticalité et l'évaluation de l'énergie cinétique du corps humain et des systèmes associant une analyse de l'actimétrie et un capteur de position.

D'une façon générale, ces différentes techniques ne sont pas efficaces 100%, mais seulement capables de détecter des chutes importantes, avec des degrés de précision variables. Il présente deux inconvénients majeurs :
- toutes les chutes ou malaises ne sont pas systématiquement détectés, en particulier lorsque le sujet est assis ou allongé ;
- ils produisent un nombre important de fausses alarmes.

On connaît par ailleurs des ensembles de surveillance médicaux non ambulatoires, capables de transmettre des mesures de certains paramètres physiologiques. Les plus sophistiqués utilisent la télémédecine. Il s'agit de systèmes complexes et coûteux, réservés à certains types de patients, mais non adaptés à des personnes âgées et/ou à mobilité réduite en site isolé.

On connaît encore différents types de capteurs physiologiques (pouls, respiration, température,...), mais ils ne sont jamais associés à des processus décisionnels, et ne permettent pas de détecter une situation anormale, telle qu'une chute.

Selon le document US- 5 515 858, on connaît également un système capable de prendre en compte d'une part des informations actimétriques et d'autre part des informations physiologiques. Le document FR-2 785 073 présente aussi un tel système, suggérant de prendre en compte les deux types d'information simultanément. Dans les deux cas, il est nécessaire de mettre en oeuvre des moyens de mesure spécifiques pour chaque type d'information.

L'invention a notamment pour objectif de pallier ces différents inconvénients des techniques antérieures.

Plus précisément, un objectif de l'invention est de fournir un dispositif et un procédé de détection de situations anormales, notamment de chutes qui présentent une fiabilité optimale (c'est-à-dire proche de 100%). En particulier, l'invention a pour objectif de permettre la détection de tous types de chute, même lorsque le sujet porteur est alité ou assis, ou a le corps appuyé contre un mur.

Un autre objectif de l'invention est de fournir un tel dispositif et un tel procédé supprimant, ou tout le moins limitant fortement, la génération de fausses alarmes (dues par exemple à un mouvement brusque, à un choc intempestif,...).

En d'autres termes, l'invention a pour objectif de permettre une détection des situations anormales qui soient à la fois plus sûre et plus efficace que les techniques connues.

Par ailleurs, un objectif de l'invention est de fournir un tel dispositif qui soit :
- relativement simple et peu coûteux à fabriquer ;
- qui présente une faible consommation, et donc une bonne autonomie;
- peu encombrant, et ergonomique (c'est-à-dire qu'il ne présente pas de gêne pour le porteur).

Ces objectifs, ainsi que d'autres qui apparaîtront par la suite, sont atteints selon l'invention à l'aide d'un dispositif de détection de situations anormales selon la revendication 1.

Ainsi, selon l'invention, on obtient une détermination efficace et précise des situations anormales, en tenant compte de deux types d'information.

Bien sûr, il est possible que, dans certaines situations, les informations d'activité soient suffisantes (chute brutale par exemple). Dans ce cas, il n'est pas obligatoire de tenir compte des informations physiologiques. Inversement, on peut prévoir que des informations physiologiques soient périodiquement relevées et analysées, même en l'absence d'une information d'activité alarmante.

Plus précisément, le dispositif de l'invention comprend :
- des moyens de détection d'une situation anormale potentielle, alimentés par un système actimétrique délivrant la ou lesdites informations d'activité ; et
- des moyens de confirmation de ladite situation anormale potentielle, alimentés par au moins un capteur physiologique délivrant la ou lesdites informations physiologiques.

Comme indiqué plus haut, il peut ne s'agir que d'un mode d'utilisation des informations d'activité et physiologiques, parmi plusieurs.

De façon préférentielle, en régime continu, seule la prise en compte d'une information actimétrique est active, et une information physiologique est prise en compte à intervalles réguliers et/ou en présence d'une situation anormale potentielle.

Cette approche permet de réaliser des systèmes à faible consommation, les informations physiologiques n'étant obtenues et analysées que lorsque cela est nécessaire pour lever un doute. En régime permanent, seules les informations d'activité sont suivies.

La technique de l'invention est donc différente des systèmes actimétriques comprenant un accéléromètre ou, avantageusement, deux ou trois accéléromètres dont les sorties sont sommées de façon à former un signal unique d'activité, alimentant lesdits moyens de détection d'une situation anormale potentielle. De tels systèmes sont notamment présentés dans les documents FR-2 785 073 et US-5 515 858 déjà mentionnés.

Selon un autre mode de réalisation avantageux, ledit système actimétrique comprend au moins un capteur de pression, assurant également la détection des paramètres physiologiques. Le signal délivré par le capteur de pression est donc riche en information, et on réalise préférentiellement une première séparation de variables, entre actimétrie et paramètres physiologiques. Une partie de l'information de mouvement peut ainsi être traitée par une interface non linéaire et servir à éveiller le microprocesseur suite à une activité motrice discontinue.

Le capteur de pression forme alors, simultanément, au moins une partie du système actimétrique et au moins une partie des capteurs physiologiques.

Préférentiellement, le ou lesdits capteurs physiologiques délivrent au moins une des informations appartenant au groupe comprenant :
- le pouls ;
- une grandeur, fonction de la pression artérielle (par exemple une pression externe fonction de la pression artérielle, modulée par la respiration, l'analyse permettant de démoduler cette grandeur et de calculer le pouls) ;
- le rythme et/ou le débit respiratoire ;
- la température (en particulier la température cutanée locale),
et les variations et/ou combinaisons de ces grandeurs.

La plupart de ces données peuvent être déduites du signal délivré par un unique capteur de pression. Ainsi, avantageusement, le dispositif de l'invention met en oeuvre au moins un capteur dynamique de pression sensible à l'impulsion cardiaque dudit sujet. On peut notamment prévoir l'agencement d'un tel capteur avec un bracelet élastique, permettant de transmettre l'impulsion cardiaque naissant à l'intérieur du poignet.

De façon avantageuse, le dispositif de l'invention comprend des moyens de mesure d'une information représentative de la force d'application dudit dispositif sur la peau dudit sujet. Cette information permet avantageusement, en outre, de vérifier si le dispositif est porté (et suffisamment bien tenu).

Selon un mode de réalisation préférentiel, il est réalisé sous la forme d'une montre bracelet. On dispose alors d'un dispositif pratique et discret.

Avantageusement, le dispositif de l'invention comprend un palpeur, ou piston, formant interface entre la peau dudit sujet et au moins un capteur physiologique. Cela permet de s'affranchir des problèmes dus à un déplacement ou un décalage de la zone d'appui sur la peau du porteur.

Selon un aspect avantageux de l'invention, le dispositif comprend des moyens de génération d'une alarme locale et des moyens de transmission vers un site distant de ladite alarme, après un délai prédéterminé, lorsque ladite alarme n'a pas été invalidée manuellement par ledit sujet.

Ainsi, on évite la transmission de fausses alertes vers un site de gestion distant. C'est le porteur qui assure lui-même la gestion des fausses alarmes.

Avantageusement, le dispositif comprend encore des moyens de déclenchement manuel, émettant une alarme vers un site distant. Le porteur peut ainsi, même en l'absence de détection automatique, avertir le site de surveillance, par exemple en cas de malaise ou de signes avant-coureurs de ce dernier.

Selon un mode de réalisation préférentiel, ledit déclenchement manuel est obtenu en appuyant fortement sur ledit dispositif, de façon à agir sur lesdits moyens de mesure d'une information représentative de la force d'application.

La mise en oeuvre est alors très simple, et ne nécessite aucun moyen supplémentaire.

De façon avantageuse, le dispositif de l'invention comprend des moyens de mémorisation d'au moins un modèle de chute et/ou d'une série desdites mesures physiologiques.

Préférentiellement, on prévoit des moyens d'affichage d'au moins une des informations appartenant au groupe comprenant :
- heure et date ;
- informations physiologiques ;
- alertes ;
- messages personnalisés, à un instant donné ;
- informations de localisation.

Le dispositif peut également comprendre au moins une photodiode, pour la transmission d'informations à des moyens de traitement dudit dispositif et/ou pour délivrer une information d'éclairement.

Le dispositif de détection de situations anormales peut également mettre en oeuvre un microphone, être associé à un système anti-fugue, comprendre des moyens de localisation et/ou des moyens de monitoring physiologique.

L'invention concerne également le procédé mis en oeuvre par le dispositif décrit ci-dessus. Ce procédé comprend notamment une étape de génération d'un signal d'alarme, représentatif d'une situation anormale, en fonction d'une analyse d'au moins une information d'activité et, au moins dans certains cas, d'au moins une information physiologique.

Préférentiellement, le procédé comprend les étapes suivantes :
- détection d'une situation anormale potentielle, en fonction de la ou desdites informations d'activité ; et
- confirmation ou infirmation de ladite situation anormale potentielle, en fonction de la ou desdites informations physiologiques.

Avantageusement, il comprend également les étapes suivantes :
- génération d'une alarme locale ;
- attente d'une commande d'annulation par ledit sujet, pendant un délai prédéterminé ;
- transmission vers un site distant de ladite alarme, lorsque ladite alarme n'a pas été annulée pendant ledit délai.

D'autres caractéristiques et avantages de l'invention apparaîtront plus clairement à la lecture de description suivante d'un mode de réalisation préférentiel de l'invention, donné à titre de simple exemple illustratif et non limitatif, et des figures annexées parmi lesquelles :
- la figure 1 est un synoptique général illustrant le principe de l'invention dans le cas d'une chute ;
- la figure 2 est un exemple de signaux délivrés par les accéléromètres en cas de chute, et de leur addition ;
- la figue 3 présente le principe du transfert de la pulsion cardiaque au dispositif de l'invention, réalisé sous la forme d'une montre bracelet ;
- les figures 4a et4b montrent un exemple d'ondes cardiaques modulées par la respiration, respectivement dans le domaine temporel et dans le domaine fréquentiel ;
- la figure 5 est un synoptique présentant les fonctionnalités du dispositif de l'invention ;
- les figures 6A et 6B présentent les différents capteurs et leur environnement mis en oeuvre pendant le dispositif de l'invention, selon deux modes de réalisation :

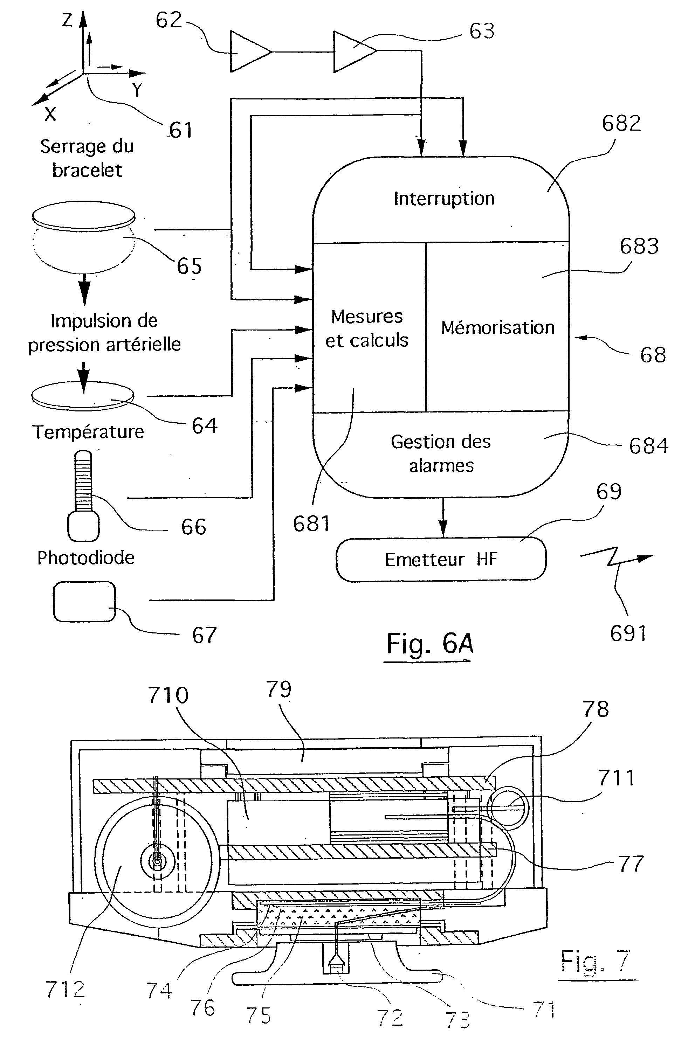

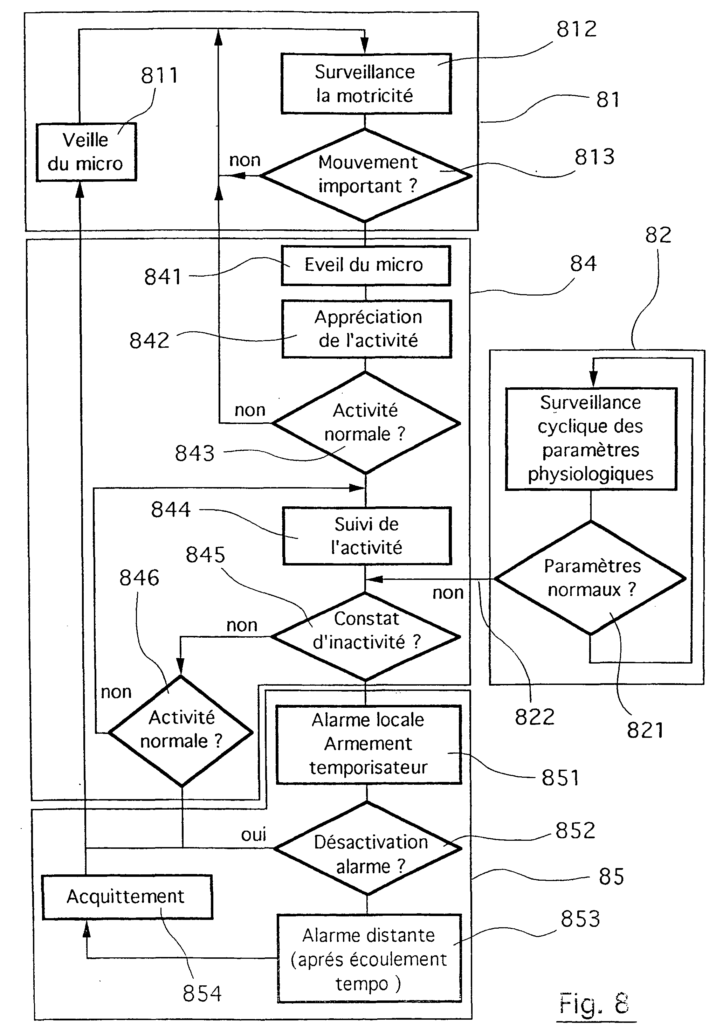

   - figure 6A : mise en oeuvre de trois capteurs piézo-électriques ;

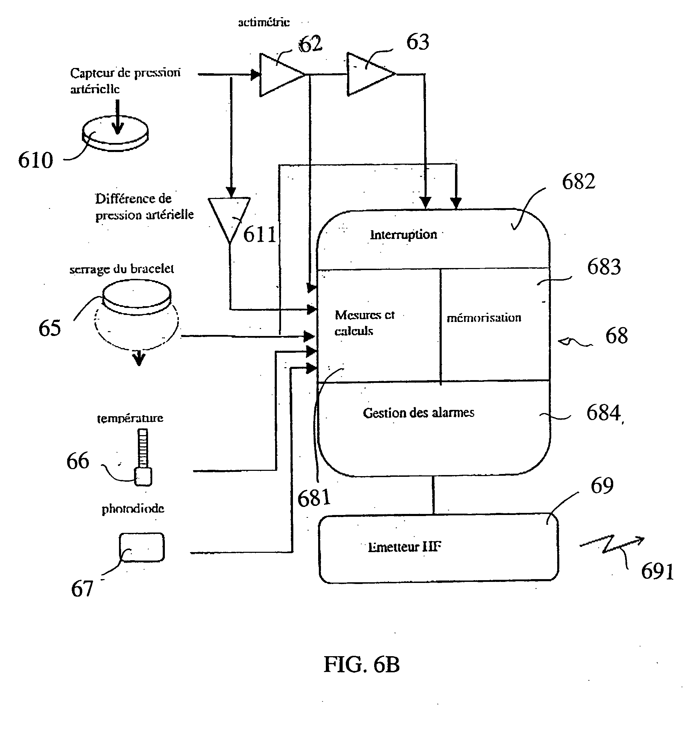

   - figure 6B : mise en oeuvre d'un capteur de pression ;
- la figure 7 montre, en coupe, un mode de réalisation du dispositif de l'invention ;
- la figure 8 est un organigramme détaillé du procédé de surveillance de l'invention.

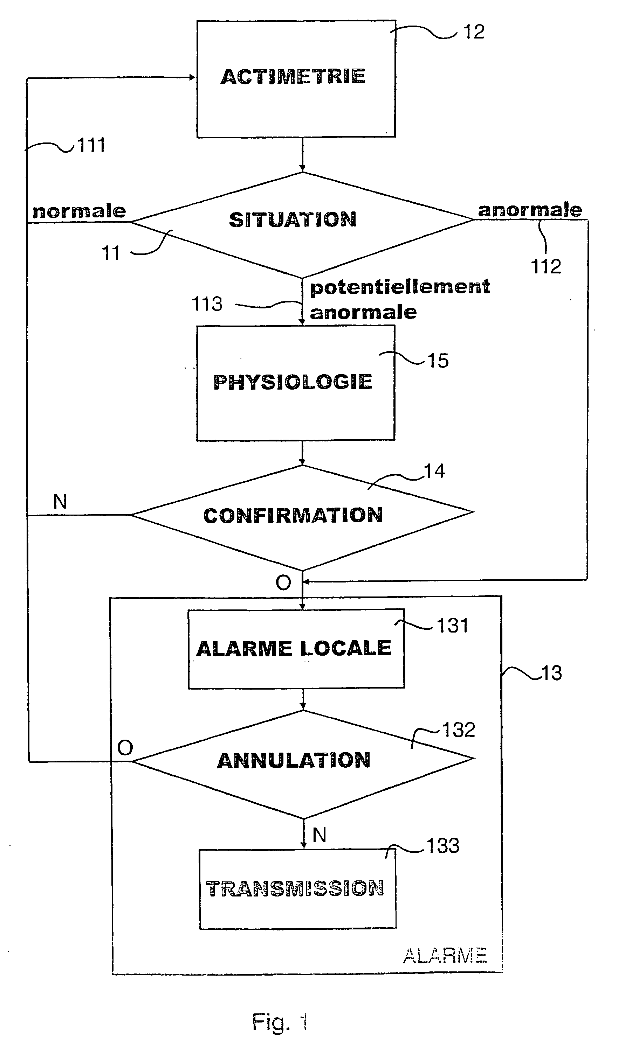

L'invention a donc pour objet un dispositif de détection de situations anormales chez un sujet, et notamment de chutes. Ce dispositif, et le procédé correspondant, sont plus fiables et plus précis que les systèmes connus, en particulier parce qu'ils combinent, au moins lors ce que cela est nécessaire, des informations actimétriques et physiologiques, ainsi que cela est illustré par le synoptique général de la figure 1.

L'analyse de la situation actimétrique du patient, en fonction de l'information actimétrique 12 peut être de trois types :
- normales 111 : seuls les capteurs actimétriques sont alors en fonction ;
- clairement anormales 112 : dans ce cas on passe directement à l'étape 13 de génération d'une alarme ;
- potentiellement anormales 113 : il s'agit du cas courant où un bougé important a été détecté, sans que l'on soit sûr qu'il s'agisse d'une chute (ce cas est d'autant plus fréquent que les seuils de détection de bouger sont faibles, de façon à détecter les "micro chutes").

Dans cette dernière situation 113, on met en oeuvre une étape supplémentaire 14 de confirmation ou d'infirmation de la normalité de la situation, en fonction d'informations actimétriques 12. Si le porteur peut bouger, il peut déclencher manuellement une alarme. Dans le cas contraire, on tient compte des informations physiologiques 15. En cas d'infirmation, on retrouve la situation normale 111. Dans le cas contraire, on passe à l'étape de génération d'une alarme. Bien entendu, les données physiologiques peuvent faire l'objet de mesures régulières, indépendamment d'une détection de mouvement, et le cas échéant provoquer l'émission d'une alarme, par exemple en cas de problème cardiaque (même s'il n'y a pas eu de chute...).

Les informations actimétriques et physiologiques peuvent également être utilisées en parallèle, ou de toute autre façon adéquate.

Selon un autre aspect avantageux de l'invention, la génération d'alarmes 13 se fait en deux temps, de façon à éviter la génération de fausses alarmes, et donc les interventions inutiles des personnes en charge de la télésurveillance. Ainsi, on génère tout d'abord une alarme locale 131, par exemple sous la forme d'un signal sonore.

Le sujet porteur dispose alors d'un laps de temps prédéterminé, par exemple quelques dizaines de secondes, pour intervenir, et annuler l'alarme s'il ne se trouve pas réellement dans une situation nécessitant une intervention. En cas d'annulation de l'alarme (132), on revient au mode normal. Dans le cas contraire, une alarme est transmise, (133) vers le site de télésurveillance.

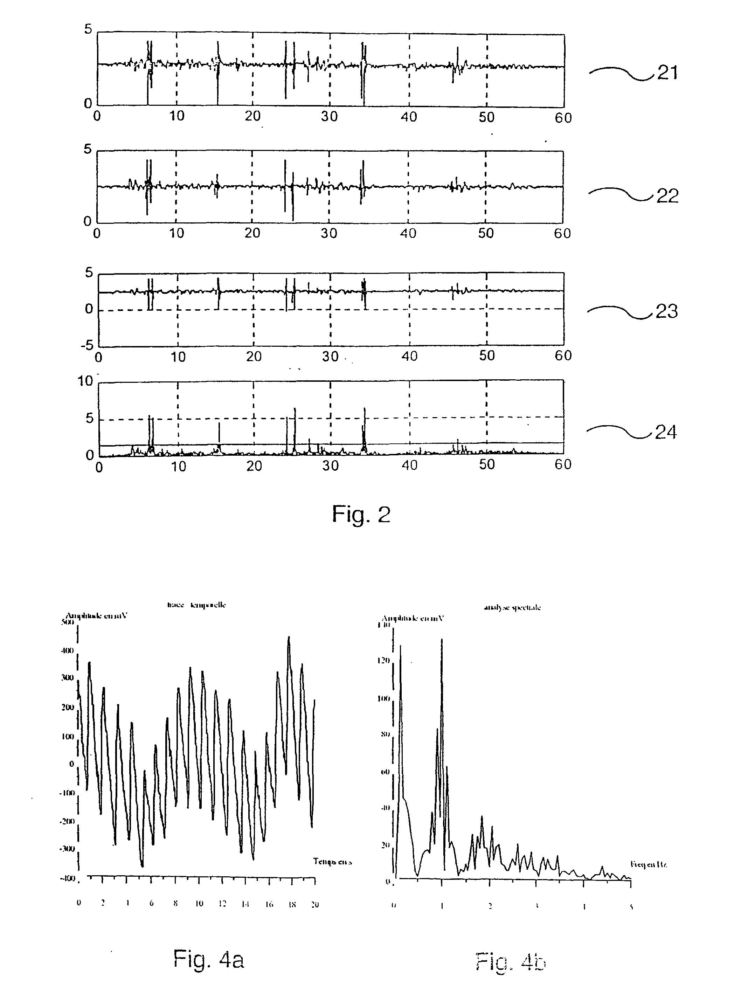

Porté avantageusement sous la forme d'un boîtier montre, le dispositif de l'invention comprend donc un ensemble de capteurs de motricité, qui permet de connaître les accélérations du poignet. Ces grandeurs, dont un exemple est donné en figure 2 (21, 22 et 23 : accélérations respectivement selon les axes X, Z et Y) servent donc en premier plan à discriminer un niveau d'énergie critique. En fait, la bande passante des accélérations lors d'une chute est comprise entre 0,5 Hz et 6 Hz, considérée comme une bande étroite.

Pour optimiser la consommation, on assimile les signaux redressés 21, 22 et 23 ajoutés et filtrés en passe-bas à l'énergie en jeu, pour obtenir le signal 24, qui sert à réveiller les moyens de calculs du dispositif.

Selon une variante avantageuse, les capteurs de motricité peuvent être remplacés par un unique capteur de pression, qui assure également la détection des paramètres physiologiques.

Dans les secondes suivantes, des mesures d'énergie sont réalisées et comparées à un modèle de chute, selon l'algorithme décrit par la suite. Après réveil, les mesures issues des accéléromètres (ou du capteur de pression, après analyse d'une partie de l'information actimétrique traitée par un interface non linéaire) sont converties en énergie. La comparaison ou le seuillage a lieu par rapport à un modèle, c'est-à-dire un ensemble de valeurs successives représentant une évolution dans le temps (par exemple la forme de l'onde après un choc), et non par rapport à un unique valeur de seuil.

Cette chute peut être relative, c'est-à-dire que le centre de gravité du corps humain reste presque invariant. Dans ce cas, la comparaison peut s'avérer délicate. C'est pour cette raison qu'on observera alors l'évolution de certains paramètres physiologiques (pouls, respiration,...) comme indicateur d'une situation anormale.

Ainsi que cela est illustré en figure 3, le dispositif boîtier-montre 31 est tenu au poignet à l'aide d'un bracelet élastique 32, similaire à celui d'une montre classique. L'impulsion cardiaque 33 traverse les vaisseaux 34, le muscle, les tissus 35 et la peau et se propagent alors à travers le bracelet 32, ce qui engendre une contrainte sur un capteur 36, décrit de façon plus détaillée par la suite.

Ainsi, les paramètres physiologiques accessibles au niveau du poignet sont l'image de l'impulsion cardiaque, dont les figures 4a et 4b donnent un exemple respectivement la trace temporelle et l'analyse spectrale correspondante d'une onde cardiaque modulée par la respiration, dans le cas d'une arythmie sinusale d'un sujet mâle de 35 ans. De ces signaux, on déduit le pouls et la fréquence respiratoire, et le cas échéant la tension artérielle.

En plaçant une sonde de température très proche de la peau, on obtient également la température locale (biaisée par rapport à la température du corps humain) de laquelle on déduit la variation de température. Sans qu'il y ait bougé, ces paramètres sont mémorisés, le système réalisant son auto-apprentissage.

Après une chute ou un malaise, le sujet peut être physiquement immobilisé. Dans ce cas, la température de son corps baisse. Ce paramètre peut être discriminant, si la variation de température du corps est significative pendant un temps suffisamment important.

On retrace l'historique des paramètres physiologiques après la chute, dans l'hypothèse où elle n'est pas détectée immédiatement (situation anormale potentielle). En fait, il est fonction de la pathologie ayant entraînée la chute. Cependant si la personne est bloquée, on peut retrouver les variations suivantes :
- pouls : bradycardie ou tachycardie ;
- respiration : fréquence en hausse suite à l'état de stress puis se régularisant, puis observation de pauses respiratoires ;
- tension artérielle : pouvant varier dans un sens comme dans l'autre (augmentation ou diminution en fonction de la cause) ;
- variation de la température : en extrapolant la réaction du corps d'une personne en situation de sommeil à celle d'une personne immobilisée, et sachant que l'homme est un endotherme puisque seule sa température interne est constante, on peut avancer que le mécanisme de thermorégulation assure le maintient de cette endothermie en dépit de conditions ambiantes pouvant être très variables. Cependant, les températures de peau ne sont pas uniformes : les températures les plus basses sont souvent celles des mains puis des pieds (28 à 31°) quand la température de la tête la plus élevée, est de l'ordre de 34 à 35°.

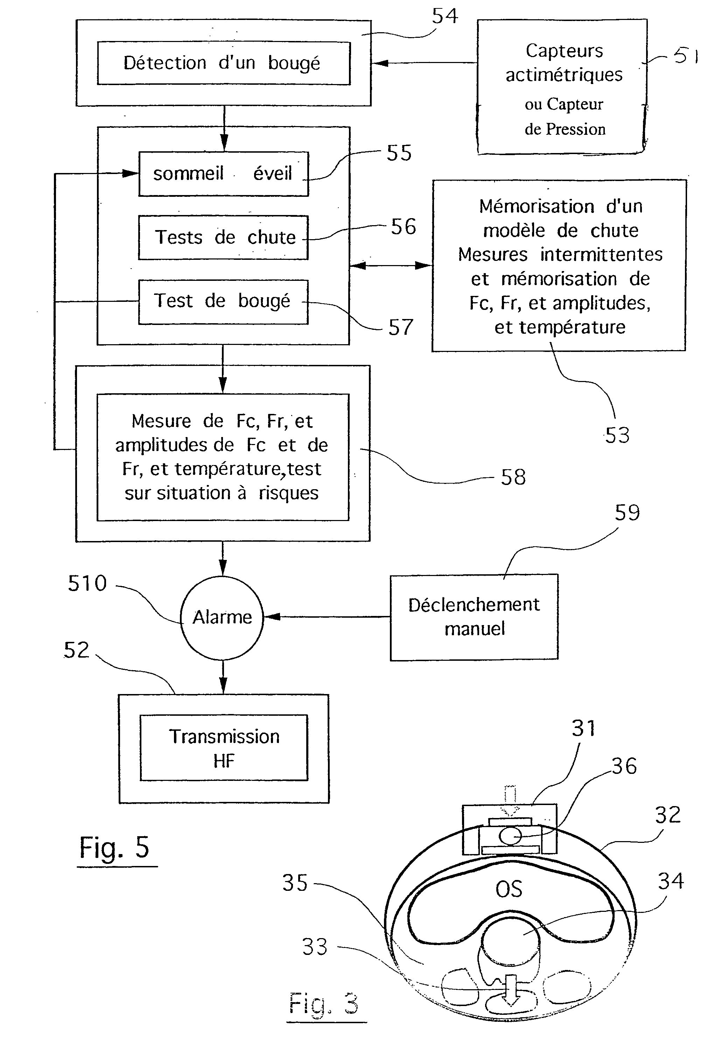

La figure 5 illustre les fonctionnalités principales du dispositif de l'invention.

En régime continu, seuls les moyens actimétriques 51 sont actifs. Le microcontrôleur est en sommeil, ainsi que l'émetteur HF. De façon intermittente, on réalise des mesures de la fréquence cardiaque (Fc) puis de la fréquence respiratoire (Fr) et de température, et des amplitudes respectives, qui sont mémorisées (53) pour suivre l'évolution de ces grandeurs, et pour éventuellement pouvoir affirmer que le sujet n'est plus en situation normale.

En cas de dépassement de niveau de la motricité (déduction d'un bougé 54) le microcontrôleur est éveillé (55), et une acquisition commence, qui a pour but de capter les signaux de motricité et de détecter s'il y a eu chute (56), à partir d'un algorithme mémorisé (53).

Puis les paramètres physiologiques précédents sont acquis (58) pendant, par exemple 30 secondes, des calculs sont réalisés est un test de dépassement opéré.

Ensuite, un test de bougé 55 est mis en oeuvre, pour vérifier que le porteur peut bouger. Dans ce cas, un déclenchement manuel 59 de l'alarme est possible, ainsi qu'une transmission d'une alarme lorsque le dispositif est non porté ou mal serré.

L'alarme 510 est transmise via des moyens de transmission HF 52. Avantageusement, la transmission précise le type d'alarme :
- montre non portée ;
- serrage déficient ;
- paramètres physiologiques hors seuil ;
- chute ;
- déclenchement manuel.

Les figures 6A et 6B présentent l'environnement des capteurs mis en oeuvre, respectivement dans le cas :
- figure 6A : mise en oeuvre de trois capteurs piézo-électriques ;
- figure 6B : mise en oeuvre d'un capteur de pression.

Dans le cas de la figure 6A, les trois accélérations sont par exemple captées par un accéléromètre 3D 61 intégré ou modulaire, par exemple du type ACH 04-08-05 fabriqué par la société AMP (marque déposée) ou tout type équivalent, ayant une sensibilité de 2 mV/g dans la bande passante 0,5 Hz-10Hz. Ils sont suivis d'un sommateur 62 (voir figure 2), d'une amplification de gain 50 et d'un seuillage 63 dans la bande passante correspondante.

La détection de la pression cardiaque est réalisée à l'aide d'un capteur piézo-électrique 64, ayant la forme d'un disque (diamètre ⌀ = 16 mm, e = 0,5 mm) utilisant l'effet piézo-électrique direct (matériau PZT PIC 155 ; construit par PI CERAMIC (marque déposée)). La valeur de la capacité pour 1 Hz est de 8,65 nF. Chargé sous une égale capacité en parallèle, avec une charge résistible de 33 MΩ, la fréquence de coupure est de 0,6 Hz et la sensibilité de 54 mV/N. un étage transimpédance assure la liaison avec un amplificateur de gain 500 et de bande passante 0,5-3Hz pour une impulsion cardiaque délivrant 1/100 de N au niveau du capteur, on peut obtenir à la sortie un signal d'amplitude crête à crête de 400 mV.

Ce capteur peut également donner l'image de l'accélération selon l'axe Z.

Dans le cas où l'on met en oeuvre un capteur de pression 610 (figure 6B), il peut s'agir avantageusement d'un capteur piezo-électrique tel que celui décrit ci-dessus. L'environnement du capteur de pression comprend une résine non élastique mais déformable formant volume entre le capteur lui-même et une membrane souple liée au piston.

Sous l'action d'un mouvement longitudinal, on retrouve le même effet que celui causé par une impulsion cardiaque. Sous une action transversale, le déplacement de la masse de la montre entraîne une variation de la longueur de bracelet d'où une variation de la contrainte détectée par le capteur de pression. Le signal émis par le capteur de pression est donc très riche en informations (information actimétrique et paramètres physiologiques).Les amplificateurs opérationnels utilisés sont du type à très faible consommation, et par exemple des amplificateurs MAX 418 de la société MAXIM (marque déposée).

Le serrage du bracelet est détecté par un capteur résistif 65 de type FSR 152-NS, capteur semi-conducteur plat recouvert d'un polymère. Intégré dans un étage à amplificateur opérationnel, il est possible de discriminer en sortie 4 niveaux qui sont :
- supérieur à 1 V : montre non portée ;
- entre 0,15 V et 0,9 V : montre portée ;
- inférieur à 0,09 V : serrage très fort, réalisée volontairement et qui sert de bouton pour l'alarme manuelle.

On notera que chacune des plages est séparée de sa voisine par une zone vierge, ce qui permet de sécuriser l'analyse.

Pour améliorer la détection de la présence de l'alarme manuelle, un test de stabilité de niveau est réalisé.

On peut également utiliser un capteur Motorola (marque déposée) MX 2300, qui améliore la discrimination des situations.

Le capteur de température 66 est peu être un capteur semi-conducteur LM 62 fabriqué par National Semiconducteur (marque déposée). Sa résolution est de 0,2°. Il se trouve avantageusement dans le palpeur, ou piston, séparé de la peau de 1 mm environ.

Le capteur photodiode 67, par exemple du type BPW 34 de SIEMENS (marque déposée), sert à transmettre des informations au moyen de calculs 681 (initialisation, tests,...), et peut informer également sur l'éclairement ambiant.

Le microcontrôleur 68 peut être décomposé, fonctionnellement, en :
- des moyens de gestion des interruptions 682, alimentés par l'information actimétrique et l'information de serrage du bracelet ;
- des moyens de mesures et de calculs 681 alimentés par les informations actimétriques, de serrage, d'impulsion de pression artérielle, de température et de la photodiode ;
- des moyens de mémorisation 683 ;
- des moyens de gestion des alarmes 684.

Dans le cas de la figure 6B, les moyens 681 et 682 sont alimentés directement par le capteur de pression artérielle 610 via les amplificateurs 62, 63 et 611 (qui délivrent une différence de pression artérielle).

Les moyens de gestion des alarmes 684 agissent sur l'émetteur HF 69, pour émettre l'alarme 691, par exemple vers une base fixe.

La figure 7 décrit l'assemblage des différents capteurs et de l'électronique intégré dans le boîtier de la montre, en coupe.

Un piston 71 est prévu pour venir en contact avec la peau du sujet porteur. Il contient la sonde de température 72. Une membrane 73, collée au piston 71, assure le transfert de l'impulsion cardiaque (ou d'un mouvement longitudinal, dans le cas du mode de réalisation à capteur de pression), permettant une variation de perpendicularité entre le piston 71 et les capteurs. La liaison entre les capteurs et le piston est faite par du gel silicone 75, découplant mécaniquement le montage.

Le capteur de détection d'impulsion cardiaque piézo-électrique 76 est collé sur le capteur de serrage de bracelet 74. Ainsi, il existe une précontrainte sur le matériau piézo-électrique, permettant la détection de petites impulsions.

L'électronique est constituée des circuits imprimés 77 et 78. Bien sûr, elle peut être réduite à un unique circuit imprimé. L'affichage de l'heure, et de toute autre information, est restituée par l'afficheur 79, par exemple du type à cristaux liquides. Les alarmes sont véhiculées via l'émetteur 710 et l'antenne 711. La source d'énergie embarquée est une pile 712, assurant une autonomie de plus d'un an au dispositif. Ce dernier est tenu au poignet par un bracelet élastique non représenté.

Les moyens d'étanchéité ne sont pas non plus représentés, mais sont choisis de façon à assurer une étanchéité au ruissellement. Les matériaux utilisés et qui sont en contact avec la peau ne sont pas blessants et plus généralement non dangereux pour un port permanent de la montre.

L'électronique comprend deux fonctions principales, qui sont l'environnement des capteurs (déjà décrits en relation avec la figure 6) et les moyens de calculs et d'émission. Les moyens de calcul embarqués peuvent être réalisés par des microcontrôleurs de très faibles consommation de la famille des MSP de Texas Instruments (marque déposée) ou de la famille des XE 88xx de XEMICS (marque déposée). Ces microcontrôleurs possèdent des moyens de mesure analogique avec une bonne précision (supérieure à 12 bits), des bases de temps, des "drivers" d'affichage pour cristaux liquides (affichage de l'heure et de certaines alarmes) une mémoire programme suffisante, et des liaisons numériques avec un émetteur HF du type FM dont la portée est de 60 m en champs libre.

Avant de décrire plus en détail l'algorithme de surveillance, on rappelle les trois points suivants :
- le déclenchement intempestif de fausses alarmes est un problème majeur pour tout système de détection automatique. Un système générant un taux de fausses alarmes importants sera un système rapidement délaissé. Pour éviter cet inconvénient, une alarme à déclenchement automatique sera systématiquement validée, sous un certain délai, par le porteur, avant d'être diffusé. L'expiration de ce délai aboutira obligatoirement à la diffusion de l'alarme, car cela laisse à supposer que le porteur est dans l'incapacité de réagir et est par conséquent en difficulté. C'est donc le porteur du système qui doit supporter les fausses alarmes, et non le service de secours ;
- une chute suivie d'une inactivité est une situation alarmante. Cet aspect est intégré pour la prise de décision finale (alerte ou non alerte) ;
- l'autonomie est un paramètre crucial pour un système ambulatoire. Il a donc été pris en compte dès le départ de la conception du dispositif. La surveillance des signaux issus des accéléromètres ou de l'information actimétrique est assurée par un dispositif analogique de faible consommation. Le rôle de ce dispositif consiste à réactiver, dans le cas d'un dépassement de seuil (activité jugée potentiellement anormale), le microcontrôleur géré en mode veille/éveil évitant ainsi toute consommation superflue. Une analyse des paramètres est alors effectuée pour déterminer si la situation est critique ou non. La surveillance des paramètres physiologiques est réalisée périodiquement de façon à déceler toute anomalie.

La figure 8 illustre l'organigramme de surveillance mis en oeuvre.

En situation normale et continue, on se trouve dans le mode de surveillance passive 81 par le dispositif analogique. Le microcontrôleur est en veille 811. On surveille la motricité 812, et on reste dans ce mode tant qu'il n'y a pas de mouvement important détecté 813.

A intervalles réguliers, on met en oeuvre la surveillance cyclique 82 des paramètres physiologiques. Tant que ces paramètres sont normaux (821), aucune action n'est engagée.

Lorsqu'un mouvement important (813) a été détecté, on passe en mode de surveillance active 84 par le dispositif analogique. On éveille le micro (841), puis on apprécie l'activité (842). Si l'activité est considérée normale (843), on revient au mode de surveillance passive. Dans le cas contraire, on passe en suivi de l'activité (844), en prenant en compte les paramètres physiologiques (822). S'il y a un constat d'inactivité, on passe au mode de système en alarme 85. Dans le cas contraire, on vérifie (846) si l'activité est normale. Si la réponse est négative, on revient à l'étape (844) de suivi de l'activité. Dans le cas contraire, le micro revient en veille (811).

Le mode alarme comprend tout d'abord une alarme locale (851), comprenant l'armement d'une temporisation. Si dans la durée de cette temporisation, l'alarme est désactivée (852), on revient à la situation normale et le micro repasse en veille (811). Dans le contraire, une alarme est transmise (853), après écoulement de la temporisation. Après acquittement (854) de l'alarme, le micro revient en veille (811).

On peut, bien entendu, prévoir de nombreuses variantes, complément et/ou option au dispositif de l'invention.

Notamment, on peut utiliser l'affichage, par exemple par cristaux liquides, pour afficher des informations tel que l'heure, ou des messages personnalisés (prise de médicaments, rendez-vous, activités,...).

Par ailleurs, dans la mesure où la bande passante de l'émetteur HF est suffisant, un microphone peut être intégré dans la montre pour transmettre la voix. On peut également prévoir des moyens de réception d'informations vocales ou autres.

Un système anti-fugue peut être réalisé, par exemple par transmission d'un signal HF toute les quinze minutes. Une fonction de détection de passage peut également être intégré via la photodiode, dans la mesure où le passage à détecter est équipé d'un émetteur lumineux. L'émission cyclique du signal HF peut également permettre de surveiller la qualité de la transmission, et donc de sécuriser le système.

La connaissance en continu du pouls, de la température et du serrage du bracelet, si l'on utilise un capteur de pression linéaire, des paramètres respiratoires,... permettent de réaliser un système de monitoring à domicile, sous la forme d'un système ambulatoire convivial.

Enfin, on peut prévoir d'associer des moyens de localisation (GPS) et/ou les fonctions de bases d'un téléphone portable, pour augmenter l'autonomie en distance et/ou constituer un système anti-fugue absolu.

## Revendications

1. Dispositif de détection de situations anormales, notamment de chutes, chez un sujet vivant, comprenant au moins un capteur de pression (36 ; 76), délivrant un signal représentatif d'une pression, et des moyens (68) de traitement d'au moins une information d'activité et d'au moins une information physiologique, **caractérisé en ce qu'**il comprend des moyens (68) dé séparation des informations présentes dans ledit signal représentatif d'une pression, délivrant auxdits moyens de traitement (68) d'une part au moins une information d'activité, représentative de l'activité dudit sujet, et d'autre part au moins une information physiologique.

2. Dispositif de détection de situations anormales selon la revendication 1, **caractérisé en ce que** lesdits moyens (68) de traitement de la ou desdites informations d'activité et de la ou desdites informations physiologiques comprennent :
- des moyens de détection (11) d'une situation anormale potentielle, alimentés par au moins une desdites informations d'activité, respectivement au moins une desdites informations physiologiques ; et
- des moyens de confirmation (14) de ladite situation anormale potentielle, alimentés par au moins une desdites informations physiologiques respectivement au moins une desdites informations d'activité, produisant une information de situation anormale confirmée commandant des moyens de génération (13) d'un signal d'alarme,
de façon à optimiser la détection des situations anormales.

3. Dispositif de détection de situations anormales selon l'une quelconque des revendications 1 et 2, **caractérisé en ce que**, en régime continu, seule la détection d'au moins une information d'activité (81) est active, et **en ce qu'**au moins une information physiologique est déterminée (82) à intervalles réguliers et/ou en présence d'une situation anormale potentielle.

4. Dispositif de détection de situations anormales selon l'une quelconque des revendications 1 à 3, **caractérisé en ce que** la ou lesdites informations physiologiques appartiennent au groupe comprenant :
- le pouls ;
- une grandeur fonction de la pression artérielle ;
- le rythme et/ou le débit respiratoire ;
et les variations et/ou combinaisons de ces grandeurs.

5. Dispositif de détection de situations anormales selon la revendication 4, **caractérisé en ce** le ou lesdits capteurs de pression comprennent au moins un capteur (36) dynamique de pression sensible à l'impulsion cardiaque dudit sujet.

6. Dispositif de détection de situations anormales selon l'une quelconque des revendications 1 à 5, **caractérisé en ce qu'**il comprend des moyens de mesure d'une information représentative de la force d'application (65) dudit dispositif sur la peau dudit sujet.

7. Dispositif de détection de situations anormales selon la revendication 6, **caractérisé en ce que** ladite information représentative de la force d'application est utilisée pour vérifier que ledit dispositif est correctement porté.

8. Dispositif de détection de situations anormales selon l'une quelconque des revendications 1 à 7, **caractérisé en ce qu'**il est réalisé sous la forme d'une montre-bracelet.

9. Dispositif de détection de situations anormales selon l'une quelconque des revendications 1 à 8, **caractérisé en ce qu'**il comprend un palpeur (71) formant interface entre la peau dudit sujet et au moins un capteur de pression.

10. Dispositif de détection de situations anormales selon l'une quelconque des revendications 1 à 9, **caractérisé en ce qu'**il comprend des moyens de génération d'une alarme locale et des moyens de transmission vers un site distant de ladite alarme, après un délai prédéterminé, lorsque ladite alarme n'a pas été invalidée manuellement par ledit sujet.

11. Dispositif de détection de situations anormales selon l'une quelconque des revendications 1 à 10, **caractérisé en ce qu'**il comprend des moyens de déclenchement manuel (59), émettant (52) une alarme vers un site distant.

12. Dispositif de détection de situations anormales selon les revendications 6 et 11, **caractérisé en ce que** ledit déclenchement manuel est obtenu en appuyant fortement sur ledit dispositif, de façon à agir sur lesdits moyens (36 ; 76) de mesure d'une information représentative de la force d'application.

13. Dispositif de détection de situations anormales selon l'une quelconque des revendications 1 à 12, **caractérisé en ce qu'**il comprend des moyens de mémorisation (683) d'au moins un modèle de chute et/ou d'une série desdites mesures physiologiques.

14. Dispositif de détection de situations anormales selon l'une quelconque des revendications 1 à 13, **caractérisé en ce qu'**il comprend des moyens d'affichage (79) d'au moins une des informations appartenant au groupe comprenant :
- heure et date ;
- informations physiologiques ;
- alertes ;
- messages personnalisés, à un instant donné ;
- informations de localisation.

15. Dispositif de détection de situations anormales selon l'une quelconque des revendications 1 à 14, **caractérisé en ce qu'**il comprend au moins une photodiode (67), pour la transmission d'informations à des moyens de traitement dudit dispositif et/ou pour délivrer une information d'éclairement.

16. Dispositif de détection de situations anormales selon l'une quelconque des revendications 1 à 15, **caractérisé en ce qu'**il comprend un microphone.

17. Dispositif de détection de situations anormales selon l'une quelconque des revendications 1 à 16, **caractérisé en ce qu'**il est associé à un système anti-fugue.

18. Dispositif de détection de situations anormales selon la revendication 17, **caractérisé en ce qu'**il comprend des moyens de localisation.

19. Dispositif de détection de situations anormales selon l'une quelconque des revendications 1 à 18, **caractérisé en ce qu'**il comprend des moyens de monitoring physiologique.

20. Dispositif de détection de situations anormales selon l'une quelconque des revendications 1 à 19, **caractérisé en ce qu'**il comprend un capteur de température (72).

21. Procédé de détection de situations anormales notamment de chutes, chez un sujet vivant, à l'aide d'au moins une information d'activité et d'au moins une information physiologique, comprenant une étape de mesure (36 ; 76) d'au moins une pression sur ledit sujet, à l'aide d'au moins un capteur de pression délivrant un signal représentatif d'une pression,
**caractérisé en ce qu'**il comprend une étape de séparation (68) des informations présentes dans ledit signal représentatif d'une pression, délivrant d'une part au moins une information d'activité, représentative de l'activité dudit sujet et d'autre part au moins une information physiologique.

22. Procédé de détection de situations anormales selon la revendication 21,
**caractérisé en ce qu'**il comprend les étapes suivantes :
- détection (11) d'une situation anormale potentielle (113), en fonction d'au moins une information d'activité ; et
- confirmation ou infirmation (14) de ladite situation anormale potentielle, en fonction d'au moins une information physiologique ;
- génération (13) d'un signal d'alarme, représentatif de la situation anormale confirmée.

23. Procédé de détection de situations anormales selon l'une quelconque des revendications 21 et 22, **caractérisé en ce qu'**il comprend les étapes suivantes :
- génération (131) d'une alarme locale ;
- attente d'une commande d'annulation (132) par ledit sujet, pendant un délai prédéterminé ;
- transmission (133) vers un site distant de ladite alarme, lorsque ladite alarme n'a pas été annulée pendant ledit délai.

24. Procédé de détection de situations anormales selon l'une quelconque des revendications 21 à 23, **caractérisé en ce qu'**au moins une desdites informations est analysée par rapport à un modèle formé d'un ensemble de valeurs successives représentatif d'une évolution dans le temps.

## Patentansprüche

1. Vorrichtung zum Erfassen ungewöhnlicher Situationen, insbesondere von Stürzen, eines lebenden Subjektes, welche mindestens einen Druckfühler (36; 76) umfasst, der ein für einen Druck repräsentatives Signal liefert, sowie Mittel (68) zum Verarbeiten von mindestens einer Aktivitätsinformation und von mindestens einer physiologischen Information,
**dadurch gekennzeichnet, dass** sie über Mittel (68) zum Trennen der in dem für einen Druck repräsentativen Signal enthaltenen Informationen verfügt, welche den Verarbeitungsmitteln (68) einerseits mindestens eine für die Aktivität des Subjektes repräsentative Aktivitätsinformation und andererseits mindestens eine physiologische Information liefert.

2. Vorrichtung zum Erfassen ungewöhnlicher Situationen nach Anspruch 1,
**dadurch gekennzeichnet, dass** die Mittel (68) zum Verarbeiten der erwähnten Aktivitätsinformation(en) und der physiologischen Information(en) Folgendes umfassen:
- Mittel zum Erfassen (11) einer potentiell ungewöhnlichen Situation, versorgt von mindestens einer der Aktivitätsinformationen bzw. mindestens einer der physiologischen Informationen, und
- Mittel zum Bestätigen (14) der potentiell ungewöhnlichen Situation, versorgt von mindestens einer der physiologischen Informationen bzw. einer der Aktivitätsinformationen, welche eine bestätigte Information für eine ungewöhnliche Situation erzeugen, die ihrerseits Mittel zum Erzeugen (13) eines Alarmsignals erzeugen,
um das Erfassen ungewöhnlicher Situationen zu optimieren.

3. Vorrichtung zum Erfassen ungewöhnlicher Situationen nach einem der Ansprüche 1 oder 2,
**dadurch gekennzeichnet, dass** bei kontinuierlichem Betrieb, nur die Erfassung einer Aktivitätsinformation (81) aktiv ist und, dass mindestens eine physiologische Information in regelmäßigen Abständen (82) und/oder beim Auftreten einer potentiell ungewöhnlichen Situation festgestellt wird.

4. Vorrichtung zum Erfassen ungewöhnlicher Situationen nach einem der Ansprüche 1 bis 3,
**dadurch gekennzeichnet, dass** die physiologische(n) Information(en) der folgenden Gruppe angehört bzw. angehören:
- dem Puls,
- einer für den Blutdruck charakteristischen Größe,
- dem Atemrhythmus und/oder dem Atmungsdurchsatz,
sowie die Variationen und/oder Kombinationen dieser Größen umfassen.

5. Vorrichtung zum Erfassen ungewöhnlicher Situationen nach Anspruch 4,
**dadurch gekennzeichnet, dass** der bzw. die Druckfühler mindestens einen dynamischen Druckfühler (36) umfasst/umfassen, der auf den Herzschlag des Subjektes reagiert.

6. Vorrichtung zum Erfassen ungewöhnlicher Situationen nach einem der Ansprüche 1 bis 5,
**dadurch gekennzeichnet, dass** sie über Mittel zum Messen einer für die Wirkungskraft (65) der Vorrichtung auf die Haut des Subjektes repräsentativen Information verfügt.

7. Vorrichtung zum Erfassen ungewöhnlicher Situationen nach Anspruch 6,
**dadurch gekennzeichnet, dass** die für die Wirkungskraft repräsentative Information dazu verwendet wird, um festzustellen, dass die Vorrichtung ordnungsgemäß getragen wird.

8. Vorrichtung zum Erfassen ungewöhnlicher Situationen nach einem der Ansprüche 1 bis 7,
**dadurch gekennzeichnet, dass** sie die Form einer Armbanduhr aufweist.

9. Vorrichtung zum Erfassen ungewöhnlicher Situationen nach einem der Ansprüche 1 bis 8,
**dadurch gekennzeichnet, dass** sie über einen Berührungsfühler (71) verfügt, der eine Schnittstelle zwischen der Haut des Subjektes und mindestens einen Druckfühler bildet.

10. Vorrichtung zum Erfassen ungewöhnlicher Situationen nach einem der Ansprüche 1 bis 9,
**dadurch gekennzeichnet, dass** sie über Mittel zum Erzeugen eines lokalen Alarms sowie über Mittel zum Senden dieses Alarms an einen fernen Ort nach einer vorgegebenen Verzögerung verfügt, wenn der Alarm nicht manuell vom Subjekt abgestellt wurde.

11. Vorrichtung zum Erfassen ungewöhnlicher Situationen nach einem der Ansprüche 1 bis 10,
**dadurch gekennzeichnet, dass** sie über Mittel zum manuellen Auslösen (59) verfügt, die einen Alarm an einen fernen Ort senden (52).

12. Vorrichtung zum Erfassen ungewöhnlicher Situationen nach einem der Ansprüche 6 und/oder 11,
**dadurch gekennzeichnet, dass** die manuelle Auslösung durch starken Druck auf die Vorrichtung entsteht, um auf die Mittel (36; 76) zum Messen einer für die Wirkungskraft repräsentativen Information einzuwirken.

13. Vorrichtung zum Erfassen ungewöhnlicher Situationen nach einem der Ansprüche 1 bis 12,
**dadurch gekennzeichnet, dass** sie über Mittel zum Speichern (683) von mindestens einem Sturzmodell und/oder einer Reihe der erwähnten physiologischen Messungen verfügt.

14. Vorrichtung zum Erfassen ungewöhnlicher Situationen nach einem der Ansprüche 1 bis 13,
**dadurch gekennzeichnet, dass** sie über Mittel zum Anzeigen (79) von mindestens einer der folgenden Informationen verfügt:
- Uhrzeit und Datum,
- physiologische Informationen,
- Alarme,
- persönliche Meldungen zu einem gegebenen Zeitpunkt,
- Ortungsinformationen.

15. Vorrichtung zum Erfassen ungewöhnlicher Situationen nach einem der Ansprüche 1 bis 14,
**dadurch gekennzeichnet, dass** sie mindestens eine Fotodiode (67) zum Senden von Informationen an Verarbeitungsmittel der Vorrichtung und/oder zum Liefern einer aufklärenden Information aufweist.

16. Vorrichtung zum Erfassen ungewöhnlicher Situationen nach einem der Ansprüche 1 bis 15,
**dadurch gekennzeichnet, dass** sie ein Mikrophon umfasst.

17. Vorrichtung zum Erfassen ungewöhnlicher Situationen nach einem der Ansprüche 1 bis 16,
**dadurch gekennzeichnet, dass** sie mit einem System zur Fluchtverhinderung verbunden ist.

18. Vorrichtung zum Erfassen ungewöhnlicher Situationen nach Anspruch 17,
**dadurch gekennzeichnet, dass** sie über Ortungsmittel verfügt.

19. Vorrichtung zum Erfassen ungewöhnlicher Situationen nach einem der Ansprüche 1 bis 18,
**dadurch gekennzeichnet, dass** sie über Mittel zur physiologischen Überwachung verfügt.

20. Vorrichtung zum Erfassen ungewöhnlicher Situationen nach einem der Ansprüche 1 bis 19,
**dadurch gekennzeichnet, dass** sie über einen Temperaturfühler (72) verfügt.

21. Verfahren zum Erfassen ungewöhnlicher Situationen, insbesondere von Stürzen, eines lebenden Subjektes, mit Hilfe von mindestens einer Aktivitätsinformation und mindestens einer physiologischen Information, die einen Schritt zum Messen (36; 76) von mindestens einem auf das Subjekt einwirkenden Druck verfügt, mit Hilfe von mindestens einem Druckfühler, der ein für einen Druck repräsentatives Signal liefert,
**dadurch gekennzeichnet, dass** es einen Schritt zum Trennen (68) der in diesem für einen Druck repräsentativen Signal vorhandenen Informationen verfügt, der einerseits mindestens eine für die Aktivität des Subjektes repräsentative Aktivitätsinformation und andererseits mindestens eine physiologische Information liefert.

22. Verfahren zum Erfassen ungewöhnlicher Situationen nach Anspruch 21,
**dadurch gekennzeichnet, dass** es die folgenden Schritte umfasst:
- Erfassung (11) einer potentiell ungewöhnlichen Situation (113), als Funktion von mindestens einer Aktivitätsinformation, und
- Bestätigung oder Außerkraftsetzung (14) der potentiell ungewöhnlichen Situation als Funktion von mindestens einer physiologischen Information,
- Erzeugung (13) eines für die bestätigte ungewöhnliche Situation repräsentativen Alarmsignals.

23. Verfahren zum Erfassen ungewöhnlicher Situationen nach einem der Ansprüche 21 und/oder 22,
**dadurch gekennzeichnet, dass** es die folgenden Schritte umfasst:
- Erzeugen (131) eines lokalen Alarms,
- Abwarten eines Befehls der Außerkraftsetzung (132) durch das Subjekt während einer vorgegebenen Verzögerung,
- Senden (133) des Alarms an einen fernen Standort, wenn der Alarm nicht innerhalb der gegebenen Zeitdauer außer Kraft gesetzt wurde.

24. Verfahren zum Erfassen ungewöhnlicher Situationen nach einem der Ansprüche 21 bis 23,
**dadurch gekennzeichnet, dass** mindestens eine der Informationen im Verhältnis zu einem Modell analysiert wird, das von einer Gruppe aufeinander folgender Werte gebildet wird, wobei diese Werte für eine zeitliche Veränderung repräsentativ sind.

## Claims

1. Device for detecting abnormal situations, particularly falls, in a living subject, comprising at least one pressure sensor (36, 76) delivering a signal representing a pressure, and means (68) for processing at least one activity-related data element and at least one physiological data element, **characterized in that** it comprises means (68) for separating the data present in the said signal representing a pressure, delivering to the said processing means (68), on the one hand, at least one activity-related data element, representing the activity of the said subject, and, on the other hand, at least one physiological data element.

2. Device for detecting abnormal situations according to Claim 1, **characterized in that** the said means (68) for processing the said activity-related data element or elements and the said physiological data element or elements comprise:
- means (11) for detecting a potential abnormal situation, supplied by at least one of the said activity-related data elements, or with at least one of the said physiological data elements; and
- means (14) for confirming the said potential abnormal situation, supplied by at least one of the said physiological data elements, or with at least one of the said activity-related data elements, producing a data element for a confirmed abnormal situation which operates means (13) for generating an alarm signal,
in such a way as to optimize the detection of the abnormal situations.

3. Device for detecting abnormal situations according to either of Claims 1 or 2, **characterized in that**, in continuous operation, only the detection of at least one activity-related data element (81) is active, and **in that** at least one physiological data element is determined (82) at regular intervals and/or in the presence of a potential abnormal situation.

4. Device for detecting abnormal situations according to any one of Claims 1 to 3, **characterized in that** the said physiological data element or elements belong to the group comprising:
- the pulse rate;
- a value which is a function of arterial pressure;
- the breathing rate and/or flow;
and variations and/or combinations of these values.

5. Device for detecting abnormal situations according to Claim 4, **characterized in that** the said pressure sensors comprise at least one dynamic pressure sensor (36) which senses the cardiac pulse of the said subject.

6. Device for detecting abnormal situations according to any one of Claims 1 to 5, **characterized in that** it comprises means for measuring a data element representing the force of application (65) of the said device to the skin of the said subject.

7. Device for detecting abnormal situations according to Claim 6, **characterized in that** the said data element representing the force of application is used to check that the said device is worn correctly.

8. Device for detecting abnormal situations according to any one of Claims 1 to 7, **characterized in that** it is made in the form of a wristwatch.

9. Device for detecting abnormal situations according to any one of Claims 1 to 8, **characterized in that** it comprises a probe (71) forming an interface between the skin of the said subject and at least one pressure sensor.

10. Device for detecting abnormal situations according to any one of Claims 1 to 9, **characterized in that** it comprises means for generating a local alarm and means for transmitting the said alarm towards a remote site, after a predetermined delay, if the said alarm has not been manually inactivated by the said subject.

11. Device for detecting abnormal situations according to any one of Claims 1 to 10, **characterized in that** it comprises manual trigger means (59) which transmit (52) an alarm towards a remote site.

12. Device for detecting abnormal situations according to Claims 6 and 11, **characterized in that** the said manual triggering is achieved by pressing hard on the said device, so as to act on the said means (36, 76) for measuring a data element representing the force of application.

13. Device for detecting abnormal situations according to any one of Claims 1 to 12, **characterized in that** it comprises means for storing (683) at least one model of a fall and/or a set of the said physiological measurements.

14. Device for detecting abnormal situations according to any one of Claims 1 to 13, **characterized in that** it comprises means (79) for displaying at least one of the data elements belonging to the group comprising:
- time and date;
- physiological data;
- alerts;
- customized messages at a given instant;
- location data.

15. Device for detecting abnormal situations according to any one of Claims 1 to 14, **characterized in that** it comprises at least one photodiode (67) for transmitting data to processing means of the said device and/or for delivering an illumination data element.

16. Device for detecting abnormal situations according to any one of Claims 1 to 15, **characterized in that** it comprises a microphone.

17. Device for detecting abnormal situations according to any one of Claims 1 to 16, **characterized in that** it is associated with an escape prevention system.

18. Device for detecting abnormal situations according to Claim 17, **characterized in that** it comprises location means.

19. Device for detecting abnormal situations according to any one of Claims 1 to 18, **characterized in that** it comprises physiological monitoring means.

20. Device for detecting abnormal situations according to any one of Claims 1 to 19, **characterized in that** it comprises a temperature sensor (72).

21. Method for detecting abnormal situations, particularly falls, in a living subject, by means of at least one activity-related data element and at least one physiological data element, comprising a step (36, 76) of measuring at least one pressure on the said subject, by means of at least one pressure sensor delivering a signal representing a pressure,
**characterized in that** it comprises a step (68) of separating the data present in the said signal representing a pressure, delivering, on the one hand, at least one activity-related data element representing the activity of the said subject and, on the other hand, at least one physiological data element.

22. Method for detecting abnormal situations according to Claim 21, **characterized in that** it comprises the following steps:
- detection (11) of a potential abnormal situation (113), based on at least one activity-related data element; and
- confirmation or negation (14) of the said potential abnormal situation, based on at least one physiological data element;
- generation (13) of an alarm signal, representing the confirmed abnormal situation.

23. Method for detecting abnormal situations according to either of Claims 21 and 22, **characterized in that** it comprises the following steps:
- generation (131) of a local alarm;
- waiting for a cancellation command (132) from the said subject, for a predetermined time;
- transmission (133) of the said alarm towards a remote site, if the said alarm has not been cancelled during the said time.

24. Method for detecting abnormal situations according to any one of Claims 21 to 23, **characterized in that** at least one of the said data elements is analysed in relation to a model constructed from a set of successive values representing a change over time.
